(19) Europäisches Patentamt
European Patent Office
Office européen des brevets

(11) Publication number: 0 195 135 B1

# (12) EUROPEAN PATENT SPECIFICATION

(45) Date of publication of patent specification: 17.06.92

(51) Int. Cl.5: C07D 215/56, A61K 31/47

(21) Application number: 85200451.4

(22) Date of filing: 21.03.85

## (54) A process for the preparation of quinoline carboxylic acid derivatives.

(43) Date of publication of application:
24.09.86 Bulletin 86/39

(45) Publication of the grant of the patent:
17.06.92 Bulletin 92/25

(84) Designated Contracting States:
AT LU NL

(56) References cited:
BE-A- 887 574

(73) Proprietor: KYORIN SEIYAKU KABUSHIKI KAISHA
2-5, Kanda Surugadai
Chiyoda-ku Tokyo(JP)

(72) Inventor: Irikura, Tsutomu
7-10-28, Oizumi Gakuen-machi
Nerima-ku, Tokyo(JP)
Inventor: Shiba, Toshie
1-7539-11, Akabane, Okaya-shi
Nagano-ken(JP)
Inventor: Matsukubo, Hiroshi
5-11-29, Minato, Okaya-shi
Nagano-jen(JP)

(74) Representative: Smulders, Theodorus A.H.J., Ir. et al
Vereenigde Octrooibureaux Nieuwe Parklaan 97
NL-2587 BN 's-Gravenhage(NL)

EP 0 195 135 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for the preparation of useful antimicrobial agents, 6,8-difluoro-1-(2-fluoroethyl)-1,4-dihydro-4-oxo-7-(1-piperazinyl or 4-methyl-1-piperazinyl)-3-quinoline-carboxylic acid having the chemical structure (IV),

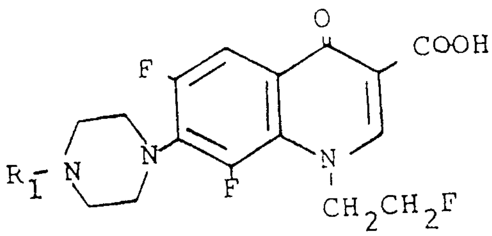

(IV)

wherein $R_1$ is a hydrogen atom or a methyl group, and more particularly, it relates to a process for the industrial manufacturing of antimicrobial agents represented by the formula (IV) having a high purity.

Such antimicrobial agents are disclosed in BE-A-887,574. The preparation method disclosed therein comprises the following steps:

(i) reaction between the ethyl ester of 6,7,8-trifluoro-1,4-dihydro-4-oxo-3-quinoline carboxylic acid and 1-bromo-2-fluoroethane;

(ii) hydrolysis to remove the ester group, which results in 6,7,8-trifluoro-1-(2-fluoroethyl)-1,4-dihydro-4-oxo-3-quinoline carboxylic acid;

(iii) reaction with piperazine or 1-methyl-piperazine to obtain the end product.

Said preparation method has several drawbacks, such as, among others, a relatively low yield (less than 20% based on the weight of the ethyl 6,7,8-trifluoro-1,4-dihydro-4-oxo-3-quinoline carboxylate starting material).

Example 15 of BE-A-887,574 teaches a method for preparing the hydrochloride salt of 6,8-difluoro-1,4-dihydro-4-oxo-7-(1-piperazinyl)-1-vinyl-3-quinoline-carboxylic acid, wherein the ethyl ester of 6,7,8-trifluoro-1,4-dihydro-4-oxo-3-quinoline carboxylic acid is reacted with 2-bromo ethanol to obtain the ethyl ester of 6,7,8-trifluoro-1,4-dihydro-1-(2-hydroxyethyl)-4-oxo-3-quinoline carboxylic acid, which is then reacted with piperazine to obtain the ethyl ester of 6,8-difluoro-1,4-dihydro-1-(2-hydroxyethyl)-4-oxo-7-(1-piperazinyl)-3-quinoline carboxylic acid. This compound is then converted into the ethyl ester of 7-(4-acetyl-1-piperazinyl)-6,8-difluoro-1,4-dihydro-1-(2-hydroxyethyl)-4-oxo-3-quinoline carboxylic acid, which in its turn is converted into the ethyl ester of 7-(4-acetyl-1-piperazinyl)-1-(2-chloroethyl)-6,8-difluoro-1,4-dihydro-4-oxo-3-quinoline carboxylic acid. Finally, this compound is subjected to a hydrolyzing treatment which removes the piperazine-nitrogen protecting acetyl group, removes the carboxyl-protecting ester group, and converts the 2-chloroethyl group into a vinyl group. In this process, all intermediates are ethyl esters and the free carboxylic group is only obtained in the last step. This example therefore shows that, at least with a compound having a 2-hydroxyethyl substituent at the quinoline-nitrogen, reaction with piperazine may be performed before removal of the ester group.

The present invention provides a process for the preparation of a compound of the formula (IV),

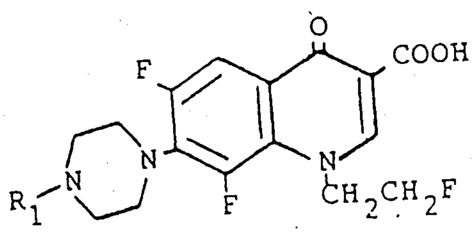

(IV)

wherein $R_1$ is a hydrogen atom or a methyl group, which comprises saponifying a compound of the formula (III),

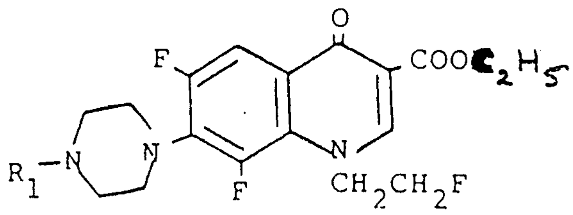

(III)

wherein $R_1$ is a hydrogen atom or a methyl group, which is prepared by the reaction of a compound of the formula (II),

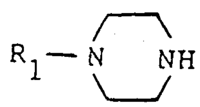

(II)

wherein $R_1$ has the above-stated meaning, with a compound of the formula (I),

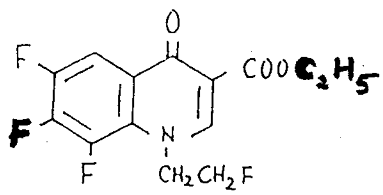

(I)

which is prepared by the reaction of a compound of the formula (V),

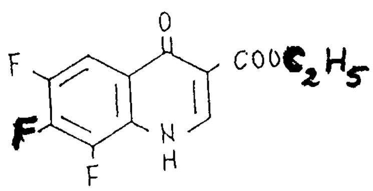

(V)

with 2-fluoroethyl tosylate.

The preparation method claimed herein comprises the following steps:

(i) reaction between the ethyl ester of 6,7,8-trifluoro-1,4-dihydro-4-oxo-3-quinoline carboxylic acid and 2-fluoroethyl tosylate;

(ii) reaction between the obtained ethyl ester of 6,7,8-trifluoro-1-(2-fluoroethyl)-1,4-dihydro-4-oxo-3-quinoline carboxylic acid and piperazine or 1-methyl-piperazine;

(iii) hydrolysis to obtain the end product.

Surprisingly, by reacting the piperazine compound with an ethyl ester of 6,7,8-trifluoro-1-(2-fluoroethyl)-1,4-dihydro-4-oxo-3-quinoline carboxylic acid and hydrolyzing the reaction product to obtain the end product, the product yield is improved significantly (above 60%) while, simultaneously, shorter reaction times, lower reaction temperatures and smaller amounts of piperazine compound may be applied.

To put it concretely, a mixture of the starting material (I) (1 mol) and piperazine derivative (II) (1 to 4 mol) is heated in a range from room temperature to 150°C, preferably 40 to 120°C, in the presence or absence of a solvent and/or base as an acceptor for the hydracid which is formed by the condensation.

A base such as, for example, pyridine, picoline, triethylamine or the like, may be used in the reaction. These organic bases may serve as the reaction solvent. Benzene, toluene, dimethyl solfoxide, dimethylformamide, acetonitrile, tert-butanol or the like may be used as the reaction solvent.

Alkyl 6,8-difluoro-1-(2-fluoroethyl)-1,4-dihydro-7-(4-methyl-1-piperazinyl)-4-oxo-3-quinolinecarboxylate (III:$R_1 = CH_3$) can also be prepared by treating alkyl 6,8-difluoro-1-(2-fluoroethyl)-1,4-dihydro-4-oxo-7-(1-piperazinyl)-3-quinolinecarboxylate (III:$R_1 = H$) with formaldehyde and a reducing agent such as formic acid.

Therefore, the present invention also provides a process for the preparation of a compound of the formula (IV-a),

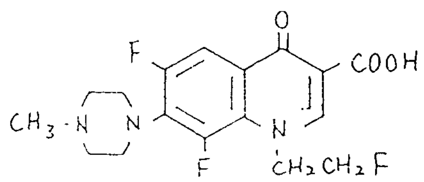

(IV-a)

which comprises saponifying a compound of the formula (III-b),

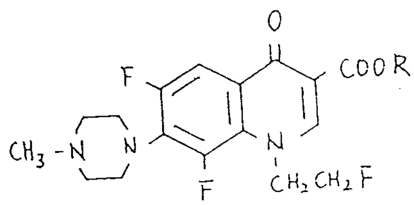

(III-b)

wherein R is a lower alkyl group having 1 to 3 carbon atoms, which is prepared by the reaction of a compound of the formula (III-a),

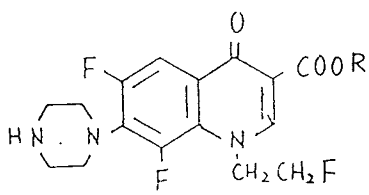

(III-a)

wherein R has the above-stated meanings, with formaldehyde and formic acid.

In the hydrolysis reaction using an acid, it is desirable that the intermediate substance (III) is heated in a mixture of mineral acid such as hydrochloric acid, sulfuric acid, and an organic solvent such as acetic acid. In the hydrolysis reaction using an alkali, the intermediate substance (III) is heated in a diluted alkali metal hydroxide such as sodium hydroxide, potassium hydroxide in a range from 40 to 100°C, preferably at 60 to 95°C.

The following examples serve to illustrate and explain the present invention.

Example 1 Ethyl 6,7,8-trifluoro-1-(2-fluoroethyl)-1,4-dihydro-4-oxo-3-quinolinecarboxylate.

To a mixture of ethyl 6,7,8-trifluoro-1,4-dihydro-4-oxo-3-quinolinecarboxylate (5.36 kg) and sodium iodide (2.94 kg) in dimethylformamide (20.5 liters), was added anhydrous potassium carbonate (2.97 kg) at 100°C under stirring.

After the addition was completed, the mixture was stirred at 95 to 100°C for 15 min. 2-Fluoroethyl tosylate (2.98 kg) was added during a period of 2 hours and the mixture was heated at 95 to 100°C for 1.5 hours. Further, 2.73 kg of 2-fluoroethyl tosylate were added during a period of 2 hours. After being heated for 4 hours, an additional 0.82 kg of the tosylate was added during a period of 1.5 hours and the reaction mixture was heated for 6 hours at the same temperature.

After cooling, the mixture was added to ice-water (64 liters). The resultant precipitate was filtered, washed with water and suspended in methanol (32 liters) under stirring for 30 min. The crystals were collected by filtration and dried to give 5.02 kg (80.0 %) of the objective compound, mp 188 - 190°C.

| | |
|---|---|
| Anal. Calcd. (%) for $C_{14}H_{11}F_4NO_3$ : | C, 53.00; H, 3.50; N, 4.42. |
| Found (%) : | C, 52.99; H, 3.40; N, 4.47. |

Example 2. Ethyl 6,8-difluoro-1-(2-fluoroethyl)-1,4-dihydro-4-oxo-7-(1-piperazinyl)-3-quinolinecarboxylate

To a hot solution (50°C) of piperazine (380 g) in dimethyl sulfoxide (840 ml) was added ethyl 6,7,8-trifluoro-1-(2-fluoroethyl)-1,4-dihydro-4-oxo-3-quinolinecarboxylate (210 g) obtained in Example 1. The reaction temperature was raised to 59°C. After 30 min, 70 g of the carboxylate was added and the mixture was maintained at 55 to 60°C for 1 hour under stirring.

Chloroform was added to the reaction mixture until it became homogeneous and then ice-water (3.5 liters) containing potassium carbonate (130 g) was added.

The mixture was agitated and the organic layer was separated. A water layer was extracted twice with chloroform. The combined chloroform layer was washed with water saturated with sodium chloride, dried over anhydrous sodium sulfate and concentrated under a reduced pressure until the crystals began to separate. Hot acetone (2 liters) was added to the mixture. The deposited crystals were collected by filtration to give the desired compound (242 g), mp 193 - 195°C. From the filtrate, a second crop (32 g) and a third crop (24 g) were obtained.

Example 3 Ethyl 6,8-difluoro-1-(2-fluoroethyl)-1,4-dihydro-7-(4-methyl-1-piperazinyl)-4-oxo-3-quinolinecarboxylate.

a) To a hot solution heated at 65°C of N-methylpiperazine (3.16 kg, 31.6 mol) in dimethyl sulfoxide (15.1 liters) was added the ester (5.02 kg, 15.8 mol.) obtained in Example 1.

The temperature of the mixture rose spontaneously to 85°C during 30 min. and was then maintained at 85 to 90°C for 1.5 hours by means of heating. After cooling, the mixture was diluted with water (35 liters) to deposit a crystalline product, which was collected by filtration and recrystallized from ethyl acetate to give 5.71 kg (90.8 %) of ethyl 6,8-difluoro-1-(2-fluoroethyl)-1,4-dihydro-7-(4-methyl)-1-piperazinyl)-4-oxo-3-quinolinecarboxylate, mp 163 - 165 ° C.

b) To a hot solution (65°C) of N-methylpiperazine (6 g, 0.06 mol.) in acetonitrile (28.5 ml) was added the ester (9.5 g, 0.03 mol.) obtained in Example 1 and the mixture was heated under reflux for 5 hours. After cooling, the mixture was diluted with 67 ml of water to deposit a crystalline product, which was recrystallized from ethyl acetate to give the objective product (10.7 g, 89.9 %), mp 160 - 163°C.

c) To a hot solution (65°C) of N-methylpiperazine (6 g) in toluene (28.5 ml) was added the ester (9.5 g) obtained in Example 1 and the mixture was heated under reflux for 4 hours. After being concentrated under a reduced pressure, the mixture was diluted with water (67 ml) to deposit crystalline product, which was recrystallized from ethyl acetate to give the objective product (7.8 g, 65.5 %), mp 159 - 162°C.

d) To a hot solution (65°C) of N-methylpiperazine (6 g) in tert. butanol (38.5 ml) was added the ester (9.5 g) obtained in Example 1 and the mixture was heated under reflux for 7 hours. After cooling, the mixture was diluted with water (67 ml) to deposit a crystalline product which was recrystallized from ethyl acetate to give the objective product (10.2 g, 85.7 %), mp 161-163°C.

e) To a hot solution (65°C) of N-methylpiperazine (6 g) in dimethylformamide (28.5 ml) was added the ester (9.5 g) obtained in Example 1. The temperature of the mixture rose spontaneously to 85°C during 30 min. and was then maintained at 85 to 90°C by heating for 7 hours. After cooling, the mixture was diluted with water (67 ml) to deposit a crystalline product, which was recrystallized from ethyl acetate to give the objective product (9.7 g, 81.5 %), mp 162 - 164°C.

Anal. Calcd. (%) for $C_{19}H_{22}F_3N_3O_3$ : C, 57.42; H, 5.58; N, 10.57.
Found (%) : C, 57.48; H, 5.49; N, 10.56.

Example 4 6,8-Difluoro-1-(2-fluoroethyl)-1,4-dihydro-7-(4-methyl-1-piperazinyl)-4-oxo-3-quinolinecarboxylic acid.

a) To a hot solution heated at 80°C of sodium hydroxide (1.81 kg) in water (61 liters) was added the ester (5.71 kg) obtained in Example 3 and the mixture was heated to 90°C for 20 to 30 min. an then at 90°C for 5 min. The mixture was brought to pH 6 by the addition of about 4 kg of 68 % acetic acid. The crystals, which precipitated from cooling, were collected by filtration and dissolved in a mixture of 68 % acetic acid (4.1 liters) and water (33 liters). The solution was treated with charcoal (0.4 kg) and filtered. To the filtrate heated at 40°C was added 35 % sulfuric acid (17.3 kg). After cooling, the precipitate (Sulfate) was collected by filtration and recrystallized from water (91 liters). The sulfate was dissolved in a solution of sodium hydroxide (1.57 kg) in water (65 liters). The solution was treated with charcoal (0.4 kg), filtered and then brought to pH 7.5 ± 0.2 by adding 68 % acetic acid (about 2.5 liters). The precipitate was filtered, and suspended in 55 liters of ethanol or methanol for 30 min. under stirring, collected by filtration and dried to give the objective product (4.48 kg, 84.4 %), mp 269.5°C.

Anal.Calcd. (%) for $C_{17}H_{18}F_3N_3O_3$ : C, 55.28;H, 4.91; N, 11.38.
Found (%) : C, 55.42; H, 4.79; N, 11.38.

b) A mixture of ethyl 6,8-difluoro-1-(2-fluoroethyl)-1,4-dihydro-4-oxo-7-(1-piperazinyl)-3-quinolinecarboxylate (297 g), formic acid (600 ml) and 39 % formalin (200 ml) was refluxed for 2 hours and then

concentrated under a reduced pressure. To the residue, water (500 ml) was added and the mixture was concentrated again. Hot water (3 liters) was added to the residue and the mixture was heated at 80°C, then treated with aqueous alkaline solution containing 160 g of sodium hydroxide under stirring. The stirring was continued until the reaction mixture became homogeneous and then further for another 10 min. Hot water (3 liters) was added and the mixture was warmed to 70 to 80°C, then neutralized with acetic acid (80 ml). Deposited crystals were collected by filtration, washed with water (3 times) and ethanol (2 times) to obtain 6,8-difluoro-1-(2-fluoroethyl)-1,4-dihydro-7-(4-methyl-1-piperazinyl)-4-oxo-3-quinolinecarboxylic acid (272 g) which was recrystallized from dimethyl sulfoxide, mp. 269°C.

Anal. Calcd. (%) for $C_{17}H_{18}F_3N_3O_3$ : C, 55.28;H, 4.91; N, 11.38.
Found (%) : C, 55.47; H. 4.82; N, 11.36.

Example 5 6,8-Difluoro-1-(2-fluoroethyl)-1,4-dihydro-4-oxo-7-(1- piperazinyl)quinoline-3-carboxylic acid.

The ester (Example 2) was saponified the same way as described in a) of Example 4.

The reaction mixture was brought to pH 7 to 8 by adding acetic acid to deposit the objective compound as the monohydrate, mp 263°C (decompd.).

Anal. Calcd. (%) for $C_{16}H_{16}F_3N_3O_3.H_2O$ : C, 51.48; H, 4.86; N, 11.26.
Found (%) : C, 51.26; H, 4.78; N, 11.26.

## Claims

**1.** A process for the preparation of a compound of the formula IV,

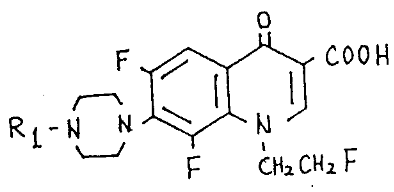

(IV)

wherein $R_1$ is a hydrogen atom or a methyl group, which comprises saponifying a compound of the formula (III),

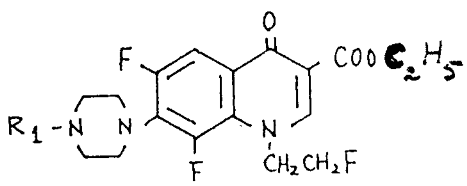

(III)

wherein $R_1$ is a hydrogen atom or a methyl group, which is prepared by the reaction of a compound of the formula (II),

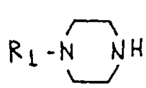

(II)

wherein $R_1$ has the above-stated meaning,
with a compound of the formula (I)

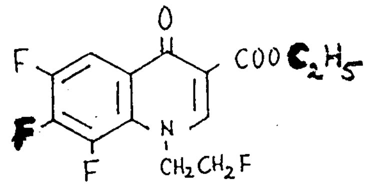

(I)

which is prepared by the reaction of a compound of the formula (V),

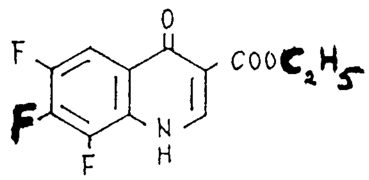

(V)

with 2-fluoroethyl tosylate.

**2.** A process for the preparation of a compound of the formula (IV-a),

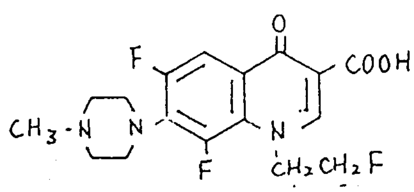

(IV-a)

which comprises saponifying a compound of the formula (III-b),

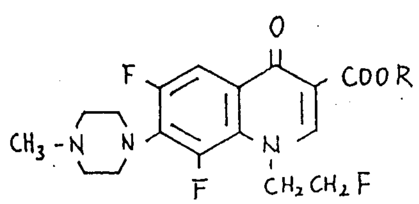

(III-b)

wherein R is a lower alkyl group having 1 to 3 carbon atoms, which is prepared by the reaction of a compound of the formula (III-a),

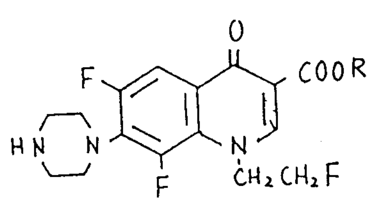

(III-a)

wherein R has the above-stated meanings, with formaldehyde and formic acid.

**Revendications**

**1.** Procédé pour la préparation d'un composé de formule (IV)

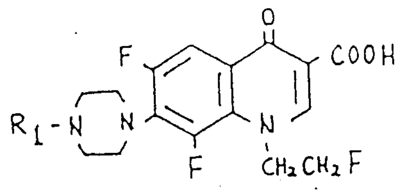

(IV)

dans laquelle $R_1$ représente un atome d'hydrogène ou un groupe méthyle, comprenant la saponification d'un composé de formule (III),

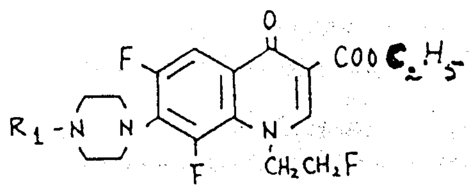

(III)

dans laquelle $R_1$ représente un atome d'hydrogène ou un groupe méthyle, qui est préparé par la réaction d'un composé de formule (II),

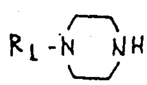

(II)

dans laquelle $R_1$ prend la signification donnée plus haut, avec un composé de formule (I)

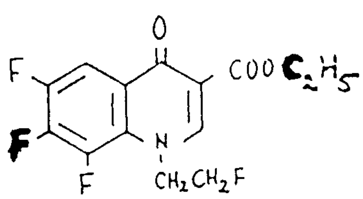

(I)

qui est préparé par la réaction d'un composé de formule (V),

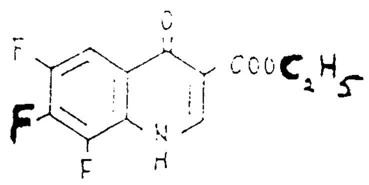

(V)

avec du 2-fluoroéthyle tosylate.

2. Procédé pour la préparation d'un composé de formule (IV-a),

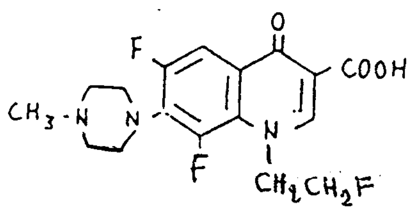

(IV-a)

comprenant la saponification d'un composé de formule (III-b),

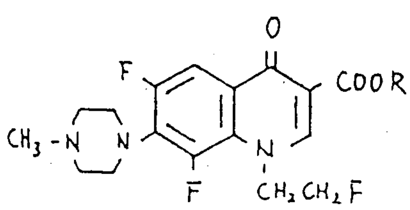

(III-b)

dans laquelle R représente un groupe alkyle à chaîne courte ayant 1 à 3 atomes de carbone, qui est préparé par la réaction d'un composé de formule (III-a),

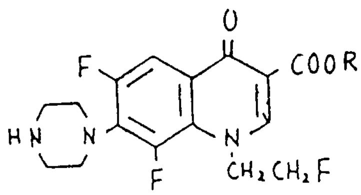

(III-a)

dans laquelle R prend la signification donnée plus haut, avec du formaldéhyde et de l'acide formique.

**Patentansprüche**

**1.** Verfahren zur Herstellung einer Verbindung der Formel (IV),

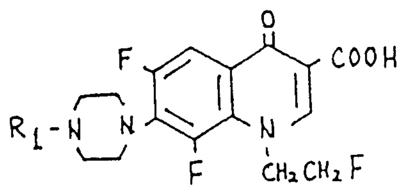

(IV)

worin $R_1$ ein Wasserstoffatom oder eine Methylgruppe ist, welches Verfahren das Verseifen einer Verbindung der Formel (III)

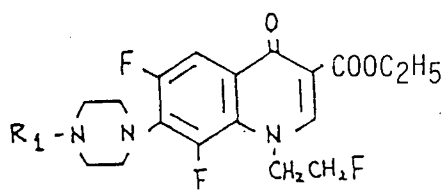

(III)

umfaßt, worin $R_1$ ein Wasserstoffatom oder eine Methylgruppe ist, welche Verbindung durch Umsetzen einer Verbindung der Formel (II)

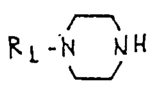

(II)

worin $R_1$ die oben angeführte Bedeutung hat,
mit einer Verbindung der Formel (I)

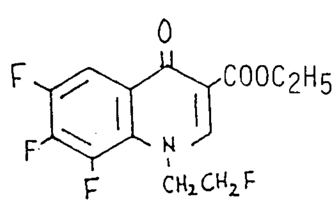

(I)

hergestellt wird, die durch das Umsetzen einer Verbindung der Formel (V)

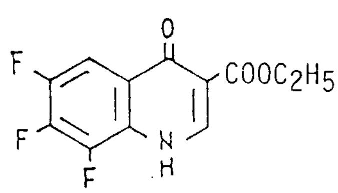

(V)

mit 2-Fluorethyltosylat hergestellt wird.

**2.** Verfahren zur Herstellung einer Verbindung der Formel (IV-a),

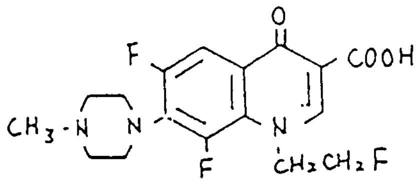

(IV-a)

welches das Verseifen einer Verbindung der Formel (III-b),

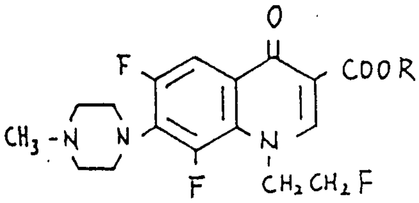

(III-b)

umfaßt, worin R eine Niederalkylgruppe mit 1 bis 3 C-Atomen ist, welche Verbindung durch das Umsetzen einer Verbindung der Formel (III-a),

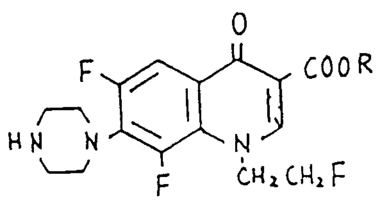

(III-a)

worin R die oben angeführte Bedeutung hat, mit Formaldehyd und Ameisensäure hergestellt wird.